# EUROPEAN PATENT APPLICATION

(11) **EP 4 159 069 A1**
(43) Date of publication of application: **05.04.2023**
(21) Application number: 20939001.2
(22) Date of filing: 25.12.2020
(51) Int. Cl.: A41D 10/00, D01F 1/10, D01F 6/62

(54) **METHOD FOR ENHANCING PARASYMPATHETIC NERVOUS SYSTEM, IMPROVING QUALITY OF SLEEP, AND RECOVERING FROM FATIGUE USING FIBER CLOTHING AND SLEEPWEAR CONTAINING PLATINUM NANOCOLLOID AND OTHER NANO PARTICLES THAT IMPROVE QUALITY OF SLEEP**

(30) Priority: 01.06.2020 JP 2020095416
(71) Applicant: Venex Co., Ltd., Atsugi-shi, Kanagawa 243-0018 (JP)
(72) Inventor: KATANO Hideki, Atsugi-shi, Kanagawa 243-0018 (JP)
(74) Representative: Vossius & Partner Patentanwälte Rechtsanwälte mbB
(86) International application number: PCT/JP2020/048937
(87) International publication number: WO 2021/245969

(57) **Abstract**

It is an object of the invention to provide a garment and sleepwear that can improve the quality of sleep, and to provide a method for improving the quality of sleep by using such a garment or sleepwear, and this is achieved by a garment for improving the quality of sleep, the garment including a fiber containing at least two kinds of nanoparticles that emit far-infrared radiation and cause electron transfer based on plasmon resonance.

## Description

### Technical Field

The present invention relates to a garment made of a fiber containing a platinum nanocolloid and other nanoparticles for improving the quality of sleep, and a method for enhancing the parasympathetic nervous system, improving the quality of sleep, and recovering from fatigue by using sleepwear.

### Background Art

It is well known that comfortableness and recovery from fatigue during sleep are related to the quality of sleep, and that good quality sleep is an important factor involved in conditioning and maintenance. In recent years, functional fibers such as a sweat-absorbing heat-generating fiber and a fiber having a cooling effect have been generally widely spread, and it is known that these functional fibers can be expected to improve comfortableness during sleep (for example, the following Non Patent Literature 1).

### Citation List

### Non Patent Literature

Non Patent Literature 1: Development of Objective Evaluation of Comfortableness for Women's Spring/Summer Pajamas, Bulletin of the Faculty of Education, Kanazawa University, 1999, 48, p. 97-109.

### Summary of Invention

### Technical Problem

It is an object of the present invention to provide a garment and sleepwear that can improve the quality of sleep, and to provide a method for improving the quality of sleep by using such a garment or sleepwear.

### Solution to Problem

The present invention relates to a garment for improving the quality of sleep, the garment including a fiber containing at least two kinds of nanoparticles that emit far-infrared radiation and cause electron transfer based on plasmon resonance. According to the present invention, the particles included in the fiber are nanoparticles and cause a phenomenon in which electrons cause collective oscillation by light (surface plasmon resonance). As a result of collective oscillation, a phenomenon in which polarization occurs in the nanoparticles and the nanoparticles are localized on the surface (localized surface plasmon resonance), may also occur. In these phenomena, it can be evaluated that the nanoparticles emit ions, and as the emitted ions, free electrons produced by localized surface plasmon resonance of the nanoparticles may be mentioned.

The particles that are included in the fiber not only have surface plasmon resonance but also have a property of emitting far-infrared radiation. According to the present invention, the quality of sleep is improved by using nanoparticles that combine these characteristic two properties. The principle for this is not specifically specified; however, a material that emits far-infrared radiation only does not produce an effect such as that of the present invention. Far-infrared radiation has a heating effect and is considered to have a sleep-inducing effect as well; however, as it is understood that taking a hot bath enhances the sympathetic nervous system and may rather disturb sleeping, the quality of sleep cannot be improved only by a heating effect. That is, by using nanoparticles that cause two phenomena, namely, far-infrared radiation and electron transfer, enhancement of the parasympathetic nervous system can be promoted, the deep body temperature is decreased, and the quality of sleep is improved.

It is preferable that at least two kinds of the above-described nanoparticles are diamond nanoparticles and platinum nanoparticles. A diamond nanoparticle and a platinum nanoparticle have an ability to emit far-infrared radiation and also efficiently cause electron transfer based on plasmon resonance, and therefore, those nanoparticles are suitable to be applied to the garment of the present invention.

Improvement of the quality of sleep can be realized by at least one of an improvement of the sleep efficiency, a decrease in the number of nocturnal awakenings, and shortening of the duration of a nocturnal awakening. As will be described in the following Examples, for example, it has been demonstrated that the number of nocturnal awakenings is significantly decreased (p value is 0.002) as compared with a garment that does not include the above-mentioned nanoparticles. The sleep efficiency and the duration of a nocturnal awakening also have remarkable effects as will be shown in the Examples.

The improvement of the quality of sleep can be realized even by at least one of an improvement of the Non-REM sleep ratio and a reduction of the REM sleep ratio. As will be shown in the following Examples, for example, the REM sleep ratio is markedly decreased (p value is 0.03) as compared with a garment that does not include the above-mentioned nanoparticles. As the REM sleep ratio is decreased, an improvement of the Non-REM sleep ratio can be promoted. Incidentally, the Non-REM sleep ratio includes the Non-REM (N1) sleep ratio, the Non-REM (N2) sleep ratio, and the Non-REM (N3) sleep ratio, and the improvement effect is applicable to all of these.

The above-described garment may be sleepwear. Since sleepwear is continuously worn while sleep, the effect of the present invention of improving the quality of sleep is sufficiently provided for a longer period of time.

The present invention also provides a method for improving the quality of sleep by using a garment including a fiber containing at least two kinds of nanoparticles that emit far-infrared radiation and cause electron transfer based on plasmon resonance. Here, using a garment includes wearing the garment.

### Advantageous Effects of Invention

According to the present invention, a garment or sleepwear that can improve the quality of sleep can be provided. Furthermore, a method for improving the quality of sleep by using such a garment or sleepwear can be provided. The improvement of the quality of sleep can also be expressed as a decrease in the deep body temperature and can also be rephrased as a method for enhancing the parasympathetic nerve activity during sleep.

### Description of Embodiments

A garment according to the present embodiment includes a fiber containing at least two kinds of nanoparticles that emit far-infrared radiation and cause electron transfer based on plasmon resonance. This fiber may be a fiber containing only two kinds of the above-mentioned nanoparticles.

Examples of such nanoparticles include a diamond nanoparticle, a platinum nanoparticle, and a particle of alumina, zirconia, silica, titanium oxide, magnesium, calcium oxide, zirconia, chromium oxide, ferrite, spinel, cerium, barium, boron carbide, silicon carbide, titanium carbide, molybdenum carbide, tungsten carbide, boron nitride, aluminum nitride, silicon nitride, zirconium nitride, carbon, graphite, tungsten, molybdenum, vanadium, tantalum, manganese, nickel, copper oxide, iron oxide, and the like. Furthermore, it is preferable that the nanoparticles consist of two kinds, namely, a diamond nanoparticle and a platinum nanoparticle.

The average particle size of the at least two kinds of nanoparticles included is suitably 10 to 200 nm. According to an embodiment of using diamond nanoparticles and platinum nanoparticles, the average particle size of the diamond nanoparticles is preferably 100 to 200 nm, and the average particle size of the platinum nanoparticles is preferably 20 to 200 nm, and more preferably 100 to 200 nm.

In a case where the fiber includes diamond nanoparticles and platinum nanoparticles, the content of the platinum nanoparticles is suitably 1/1000 to 1 times the content of the diamond nanoparticles. As a method for incorporating nanoparticles of the fiber, a method of spinning with a fiber material containing the nanoparticles, a method of adhering the nanoparticles to a spun fiber, and the like may be mentioned.

As the diamond nanoparticles (nano-sized diamonds), rough diamonds synthesized by a detonation method (hereinafter, also referred to as blended diamonds or BD) and GD (Generated Diamonds) obtainable by producing BD may also be used.

Rough diamonds produced by a detonation method are aggregates which are formed from diamond and graphite having a diameter of several ten to several hundred nanometers (nm), and in which very small nanocluster-sized diamond units (nanodiamonds) having a diameter of 1.7 to 7 nm are firmly aggregated. That is, the rough diamonds are firm aggregates of at least four, usually several ten to several hundred pieces, and in some cases, several thousand pieces of nanodiamonds. It is presumed that the particles of BD have a core-shell structure in which graphite-based carbon covers the surface of diamond, and a large number of hydrophilic functional groups such as -COOH and -OH are present on the surface of graphite-based carbon so that the particles of BD have a very satisfactory affinity with a solvent having an -OH group, such as water, alcohol, or ethylene glycol, and are rapidly dispersed in these solvents. Above all, the dispersibility in water is the most satisfactory. BD contains a very small amount of minute (1.5 nm or less) amorphous diamonds, and graphite and non-graphite carbon ultrafine particles.

The specific gravity of the nano-sized diamond particles is preferably 2.50 to 3.45 g/cm³, more preferably 2.63 to 3.38 g/cm³, and most preferably 2.75 to 3.25 g/cm³. The specific gravity of the nano-sized diamonds is determined by the ratio between graphite and diamond, and when the ratio of diamond and graphite is calculated by taking the specific gravity of diamond as 3.50 g/cm³ and the specific gravity of graphite as 2.25 g/cm³, a specific gravity of 2.63 g/cm³ corresponds to a composition of 30% by volume of diamond and 70% by volume of graphite, while a specific gravity of 3.38 g/cm³ corresponds to a composition of 90% by volume of diamond and 10% by volume of graphite. Similarly, a specific gravity of 2.75 g/cm³ corresponds to a composition of 40% by volume of diamond and 60% by volume of graphite, and a specific gravity of 3.25 g/cm³ corresponds to a composition of 80% by volume of diamond and 20% by volume of graphite. Furthermore, a specific gravity of 2.87 g/cm³ corresponds to a composition of 50% by volume of diamond and 50% by volume of graphite. When the specific gravity is less than 2.63 g/cm³, coloration caused by graphite may be problematic, and when the specific gravity is more than 3.38 g/cm³, since the far-infrared radiation emission effect is saturated, it is disadvantageous in terms of cost.

Impurities of BD can be divided into: (i) water-soluble electrolytes (ionized); (ii) hydrolyzable groups and ionic substances (salts of functional surface groups and the like) that are hydrogen-bonded to the surface of diamond; (iii) water-insoluble substances (impurities, insoluble salts, and insoluble oxides attached to the surface); (iv) volatile substances; and (v) substances incorporated or encapsulated in the diamond crystal lattice.

The items (i) and (ii) are formed in a purification process for GD. The water-soluble electrolytes of (i) can be removed by washing with water; however, in order to remove the water-soluble electrolytes more effectively, it is preferable to treat the water-soluble electrolytes with an ion-exchange resin. The water-insoluble impurities of (iii) are composed of separated microparticles such as metals, metal oxides, metal carbides, and metal salts (sulfates, silicates, and carbonates); non-separable surface salts; surface metal oxides; and the like. In order to remove these, it is preferable to change them into a soluble form by means of acid. The volatile impurities of (iv) can be removed by heat-treating the impurities at 250°C to 400°C, usually in a vacuum of about 0.01 Pa.

In the nano-sized diamonds, it is not necessarily essential to completely remove impurities; however, it is preferable to remove 40% to 95% of the impurities of (i) to (iii). The ratio of graphite and diamond can be regulated by changing the conditions for the detonation method and/or changing the purification conditions for BD.

A dispersion of the nano-sized diamonds is produced by subjecting a diamond-non-diamond mixture (initial BD) obtained by a detonation of an explosive to an oxidation treatment, subsequently adding a volatile material or a basic material whose decomposition reaction product becomes volatile so as to neutralize the diamond-non-diamond mixture, and separating a layer containing diamond.

An oxidation treatment step consists of an oxidative decomposition treatment by means of nitric acid, and an oxidative etching treatment by means of nitric acid conducted thereafter. The oxidative etching treatment consists of a primary oxidative etching treatment and a secondary oxidative etching treatment, and it is preferable that the primary oxidative etching treatment is carried out at a pressure and a temperature higher than the pressure and temperature of the oxidative decomposition treatment, while it is preferable that the secondary oxidative etching treatment is carried out at a pressure and a temperature higher than the pressure and temperature of the primary oxidative etching treatment. It is preferable that the oxidative treatment step is carried out several times at 150°C to 250°C and 14 to 25 atmospheres for at least 10 to 30 minutes each time.

After the oxidative etching treatment, a neutralization treatment for decomposing and removing nitric acid is carried out. The dispersion liquid neutralized by the basic material is separated into a phase containing diamond and a phase that does not contain diamond, by adding water to the dispersion liquid and inclining the dispersion liquid.

After the phase containing diamond is separated, nitric acid is further added to the dispersion liquid to perform a washing treatment, and a treatment of separating the lower phase dispersion liquid including the produced diamond microparticles from the upper phase effluent is carried out. This treatment for separation is carried out by leaving the dispersion liquid after a nitric acid washing treatment to stand still.

With regard to the lower phase dispersion liquid including the produced diamond microparticles, the pH is regulated preferably to 4 to 10, more preferably 5 to 8, and most preferably 6 to 7.5, and the diamond microparticle concentration is adjusted preferably to 0.05% to 16% by mass, more preferably 0.1% to 12% by mass, and most preferably 1% to 10% by mass.

The GD obtainable in this manner has an elemental composition with 72% to 89.5% of carbon, 0.8% to 1.5% of hydrogen, 1.5% to 2.5% of nitrogen, and 10.5% to 25% of oxygen. In the entire amount of carbon, 90% to 97% is diamond crystals, and 10% to 3% is non-diamond carbon. The average particle size (primary particles) is 2 to 50 nm. In an X-ray diffraction spectrum (XD) obtained by using Cu and Kα rays as the radiation source, there is the strongest peak at a Bragg angle (2Φ ± 0.2°) of 43.9°, there are characteristic strong peaks at 73.5° and 95°, there is a highly localized halo at 17°, and there is substantially no peak at 26.5°. Furthermore, the specific surface area is 1.5×10⁵ m²/kg or more, substantially all the surface carbon atoms are bonded to heteroatoms, and the dispersion liquid contains 0.05 to 16 parts by mass of diamond particles having a total absorption space of 0.5 m³/kg or more. The particle size of the GD particles is obtained by dynamic light scattering measurement using an electrophoretic light scattering photometer model ELS-8000.

The platinum nanoparticles can be obtained as a platinum nanocolloid by, for example, a method described below.

That is, the method includes a step of adding a peptide or a glucosamine compound as compound A represented by Formula (1) or Formula (2) as a reducing agent, into a solution in which a noble metal-containing compound containing platinum is dissolved; a step of adding an alkali that assists the reducing property of the compound A; and a step of forming noble metal microparticles (platinum microparticles) by a reduction reaction of noble metal ions in the noble metal-containing compound.

NR⁴R⁵-CR²R³-CO-R¹ ... (1)

NR⁴R⁵-CR⁶R⁷-CR²R³-CO-R¹ ... (2)

Here, R¹ represents an atomic group bonded by hydrogen, a hydroxyl group, an alkoxy group, an amino group, or a peptide bond; R² and R³ each represent hydrogen, an alkyl group, or a substituted alkyl group; R⁴ and R⁵ each represent hydrogen, an alkyl group, a substituted alkyl group, or an acetyl group; and R⁶ and R⁷ each represent hydrogen, an alkyl group, or a substituted alkyl group.

In the above-described production method, the solution is suitably an aqueous solution. More suitably, in the step of adding an alkali, the alkali is added such that the pH of the aqueous solution is 10 or higher. Even more suitably, in the step of forming noble metal microparticles, a noble metal colloid in which the noble metal microparticles are dispersed in the aqueous solution is produced. Still more suitably, after the step of forming noble metal microparticles and producing a noble metal colloid, the production method further includes a step of centrifuging the aqueous solution to separate the aqueous solution into a sediment and a supernatant, and a step of removing the supernatant to take out the sediment.

According to another suitable embodiment of the above-described production method, the solution is a solution of an organic solvent. More suitably, after the step of forming noble metal microparticles, the production method further includes a step of leaving the solution to stand to separate the solution into a sediment and a supernatant, and a step of removing the supernatant to take out the sediment. Even more suitably, after the step of removing the supernatant to take out the sediment, the production method further includes a step of adding water to the sediment to produce a water-based noble metal colloid. Still more suitably, after the step of producing the noble metal colloid, the production method further includes a step of concentrating the noble metal colloid by ultrafiltration, ultracentrifugation, or the like. Even more suitably, the production method further includes a step of adding a protective agent for an organic solvent into the solution, and after the step of removing the supernatant to take out the precipitate, the production method further includes a step of adding an organic solvent to the sediment to produce an organic solvent-based noble metal colloid.

Examples of the compound A as a peptide include an α- or β-amino acid compound such as an α- or β-amino acid in which R1 in Formula (1) or (2) is a hydroxyl group, or an α- or β-amino acid ester in which R¹ represents an alkoxy group; an α- or β-amino acid compound in which the amino group is acetylated; and a peptide in which R¹ is an atomic group bonded with a peptide bond, and the N-terminus is an α-amino acid.

Examples of the compound A as a glucosamine compound include glucosamine compounds such as glucosamine in which R¹ in Formula (1) is hydrogen, R² is [-CH(OH)-CH(OH)-CH(OH)-CH₂(OH)], and R³, R⁴, and R⁵ are each hydrogen; or N-acetylglucosamine, which is a derivative of glucosamine.

As a method for producing a fiber containing at least two kinds of nanoparticles that emit far-infrared radiation and cause electron transfer based on plasmon resonance, a method of preparing a composition (spinning dope) containing at least two kinds of nanoparticles having the above-described properties and producing a fiber from the composition by a wet or dry spinning method, may be mentioned.

The above-described composition may contain a polymer such as a polyester, in addition to at least two kinds of nanoparticles that emit far-infrared radiation and cause electron transfer based on plasmon resonance, and may also contain water, an alcohol, an organic solvent, or the like as a medium. Furthermore, the composition may also contain a dispersion stabilizer or the like to the extent that does not impair the effect of the present invention. As the medium, water is preferred.

The fiber according to the present embodiment is preferably a synthetic fiber such as a polyester fiber.

A garment according to the present embodiment may include a fiber containing at least two kinds of nanoparticles that emit far-infrared radiation and cause electron transfer based on plasmon resonance, and may further include another fiber. Examples of the other fiber include synthetic fibers such as a polyester fiber, nylon, and acryl; natural fibers such as cotton, hemp, and silk; and regenerated fibers such as rayon, Tencel, and lyocell, which do not contain the above-described nanoparticles.

The content of the fiber containing at least two kinds of the above-mentioned nanoparticles, which is included in the garment according to the present embodiment, may be, for example, 10% by mass or more, may be 20% by mass or more, may be 100 by mass or less, may be 80% by mass or less, may be 60% by mass or less, or may be 50% by mass or less.

Such a garment according to the present embodiment can be used as, for example, a housecoat, an indoor gown, or the like, and the sleepwear can be used as, for example, a pajama, a bathrobe (light cotton kimono), or sheet-like nightgown, so that the quality of sleep can be improved.

By wearing the above-mentioned garment, a method for improving the quality of sleep can be provided.

This method can also be expressed as a method for improving the quality of sleep, the method including wearing a garment including a fiber containing at least two kinds of nanoparticles that emit far-infrared radiation and cause electron transfer based on plasmon resonance, the method including at least one of (1) an increase in the sleep efficiency value; (2) a decrease in the number of nocturnal awakenings; (3) a decrease in the duration of a nocturnal awakening; (4) a decrease in the light sleep ratio (for example, REM sleep ratio); and (5) an increase in the deep sleep ratio (for example, Non-REM sleep ratio). Furthermore, this method can also be rephrased as a method for enhancing the parasympathetic nerve activity during sleep.

### Examples

Hereinafter, the present invention will be described in more detail by way of Examples and Comparative Examples. However, the present invention is not intended to be limited to the following Examples.

### [Production of garment]

### (Example 1)

A polyester fiber including 0.01 mg/kg or more of diamond nanoparticles (ND) and 0.0001 mg/kg or more of platinum nanoparticles (NP) was produced. The nanoparticles in this polyester fiber included platinum nanoparticles having an average particle size of 20 nm and a Round shape (circular shape) and diamond nanoparticles having an average particle size of 120 nm and an Oval shape (elliptical shape). The obtained polyester fiber at a proportion of 20% by mass and a general polyester fiber that did not contain the above-described particles at a proportion of 80% by mass were mixed, and sleepwear (a short-sleeved shirt and long pants) was produced (test garments).

### (Comparative Example 1)

Sleepwear (a short-sleeved shirt and long pants) was produced (target garments) by using a general polyester fiber that did not contain the above-described particles.

### [Evaluation of quality of sleep]

Fourteen normal healthy men who were given explanations of the contents of the present study and agreed to participate, were subjected to evaluation. Each of the test subjects was asked to wear the sleepwear of Example 1 and Comparative Example 1, a wearable heart rate variation measuring instrument (trade name: Silmee, manufactured by TDK Corporation) was attached to the chest with gel pads, the variation data of the heart rate was acquired, and then the quality of sleep (sleep efficiency, the number of nocturnal awakenings, and the duration of a nocturnal awakening) was automatically analyzed with dedicated software based on the acquired data by using a PC. The results are presented in Table 1. In Table 1, regarding the sleep efficiency, a higher value indicates that the quality of sleep is higher, and regarding the number of nocturnal awakenings and the duration of a nocturnal awakening, lower values indicate that the parasympathetic nerve activity is active and good-quality sleep is obtained.

**[Table 1]**

| | Example 1 (n = 14) | Comparative Example 1 (n = 14) | p value |
|---|---|---|---|
| Sleep efficiency | 0.93 ± 0.04 | 0.90 ± 0.04 | 0.002 |
| Number of nocturnal awakenings | 8.22 ± 4.67 | 10.63 ± 5.14 | 0.002 |
| Duration of nocturnal awakening | 29.51 ± 17.01 | 41.17 ± 22.50 | 0.003 |

Twelve normal healthy persons who were given explanations of the contents of the present study and agreed to participate, were subjected to evaluation. Each of the test subjects was asked to wear the sleepwear of Example 1 and Comparative Example 1, the respective sensors for brain waves, eyeball movement, expiratory flow rate, and snoring, the electromyography electrodes, and the electrocardiogram electrodes were installed for an overnight sleep polygraph examination, the data of the sleep state was acquired overnight, and then the acquired data were classified into four classes from light sleep to deep sleep (REM, Non-REM (N1), Non-REM (N2), and Non-REM (N3)). The results are shown in the following Table 2. In Table 2, when the sleepwear of Example 1 was worn, the REM sleep ratio (%) indicated a low ratio. From this, it was shown that when the sleepwear of Example 1 was worn, the duration of light sleep during sleep was short, while the duration of deep sleep (N1, N2, and N3) was long, and this indicates that the parasympathetic nerve activity was active, and that good-quality sleep was obtained.

**[Table 2]**

| Sleep ratio | Example 1 (n = 12) | Comparative Example 1 (n = 12) | p value |
|---|---|---|---|
| N1 | 8.28 ± 3.49 | 7.66 ± 2.76 | 0.31 |
| N2 | 54.14 ± 6.91 | 54.3 ± 10 | 0.81 |
| N3 | 14.43 ± 6.23 | 12.36 ± 5.33 | 0.45 |
| REM | 23.13 ± 4.46 | 25.78 ± 4.61 | 0.03 |

From the above results, it was found that when the sleepwear of Example 1 was worn during sleep, the parasympathetic nerve activity during sleep was enhanced, nocturnal awakening was reduced, and the sleep efficiency was increased, so that the quality of sleep was increased, which affected recovery from fatigue and performance improvement.

## Claims

1. A garment for improving a quality of sleep, the garment comprising a fiber containing at least two kinds of nanoparticles that emit far-infrared radiation and cause electron transfer based on plasmon resonance.

2. The garment according to claim 1, wherein at least two kinds of the nanoparticles are diamond nanoparticles and platinum nanoparticles.

3. The garment according to claim 1 or 2, wherein the quality of sleep is improved by at least one of an improvement of sleep efficiency, a decrease in the number of nocturnal awakenings, and shortening of a duration of a nocturnal awakening.

4. The garment according to any one of claims 1 to 3, wherein the quality of sleep is improved by at least one of an improvement of a Non-REM sleep ratio and a decrease in a REM sleep ratio.

5. The garment according to any one of claims 1 to 4, wherein the garment is sleepwear.

6. A method for improving a quality of sleep, the method comprising wearing a garment including a fiber containing at least two kinds of nanoparticles that emit far-infrared radiation and cause electron transfer based on plasmon resonance.

7. The method according to claim 6, wherein at least two kinds of the nanoparticles are a diamond nanoparticle and a platinum nanoparticle.

8. The method according to claim 6 or 7, wherein the quality of sleep is improved by at least one of an improvement of a sleep efficiency, a decrease in the number of nocturnal awakenings, and shortening of a duration of a nocturnal awakening.

9. The method according to any one of claims 6 to 8, wherein the quality of sleep is improved by at least one of an improvement of a Non-REM sleep ratio and a decrease in a REM sleep ratio.
